## Europäisches Patentamt
## European Patent Office
## Office européen des brevets

(11) Veröffentlichungsnummer: **0 128 390**
**B1**

# (12) EUROPÄISCHE PATENTSCHRIFT

(45) Veröffentlichungstag der Patentschrift:
08.04.87

(21) Anmeldenummer: **84105586.6**

(22) Anmeldetag: **16.05.84**

(51) Int. Cl.⁴: **A 61 B 5/10**

## (54) Vorrichtung zur Erfassung der Drehfähigkeit der Halswirbelsäule.

(30) Priorität: **17.05.83 DE 3317918**

(43) Veröffentlichungstag der Anmeldung:
**19.12.84 Patentblatt 84/51**

(45) Bekanntmachung des Hinweises auf die Patenterteilung:
**08.04.87 Patentblatt 87/15**

(84) Benannte Vertragsstaaten:
**AT CH DE FR GB IT LI NL SE**

(56) Entgegenhaltungen:
**FR-A-941 542**
**US-A-2 219 999**
**US-A-2 565 381**
**US-A-2 670 729**
**US-A-3 520 293**
**US-A-3 610 227**

(73) Patentinhaber: **Gross, Gisela Herma Ottilie, Wilhelm- Leuschner- Strasse 11, D-6078 Neu-Isenburg (DE)**

(72) Erfinder: **Gross, Dieter, Dr., verstorben, D-Frankfurt/Main (DE)**

(74) Vertreter: **Weickmann, Heinrich, Dipl.- Ing., Patentanwälte Dipl.- Ing. H.Weickmann Dipl.- Phys.Dr. K.Fincke Dipl.- Ing. F.A.Weickmann Dipl.- Chem. B. Huber Dr.- Ing. H. Liska Dipl.- Phys.Dr. J. Prechtel Postfach 860820, D-8000 München 86 (DE)**

## Beschreibung

Die Erfindung betrifft eine Vorrichtung zur Erfassung der Drehfähigkeit der Halswirbelsäule.

Eine solche Vorrichtung ist aus der Zeitschrift "Orthopädie", 89. Band, 1958, Heft 4, Seiten 557 und 558, bekannt.

Die bekannte Vorrichtung umfaßt einen Trägerstab mit zwei Schulterauflagen, wobei der Trägerstab bei Auflage der Schulterauflagen auf den Schultern eines Patienten hinter dem Nacken des Patienten vorbeiläuft. Die Anlage der Vorrichtung an dem Patienten erfolgt während der Patient steht. An dem Trägerstab ist ein kreisbogenförmiger Skalentrager angebracht, der in Kinnhöhe vor dem Patienten vorbeiläuft. Die Messung der Drehfähigkeit erfolgt dadurch, daß die maximal möglichen Ausschläge des Kinns des Patienten auf der Skala abgemessen werden. Die Vorrichtung ist zwar ein nutzlicher Behelf, jedoch erlaubt sie keine genaue Messung. In aufrechter Körperhaltung stören die Stell- und Haltereflexe der Nackenmuskulatur. Die Kopfdrehung ist deswegen kleiner als im Liegen, wo diese Stell- und Haltereflexe nicht wirken. Die Bezugspunkte sind überdies sehr ungenau definiert. Die Schulterstellung variiert durch die Haltung der Person und durch Veränderungen des gesamten Stütz- und Bewegungsapparates, z.B. durch einen Buckel. Ferner hat das Kinn keine definierte Spitze. Die Meßstelle bleibt damit auch variabel. Schließlich wird das Meßergebnis bei der Messung nicht unmittelbar registriert und unterliegt somit Ablese- und Übertragungsfehlern.

Der Erfindung liegt die Aufgabe zugrunde, eine Vorrichtung anzugeben, die es ermöglicht, die Drehfähigkeit der Halswirbelsäule nach beiden Seiten genau reproduzierbar zu ermitteln, um den Einfluß von therapeutischen Maßnahmen auf die Drehfähigkeit objektiv ermitteln zu können.

Zur Lösung dieser Aufgabe werden erfindungsgemäß die Maßnahmen nach dem kennzeichnenden Teil des Anspruchs 1 vorgesehen.

Die erfindungsgemäße Vorrichtung erlaubt auf der Basis einer genauen Rotationserfassung eine einwandfreie Dokumentation; so ist es z.B. möglich, eine posttraumatische Einschränkung der Drehung zu ermitteln; weiter ist es möglich, das Ergebnis einer manuellen Therapie objektiv festzustellen. Deshalb ist die erfindungsgemäße Vorrichtung von Bedeutung für die Diagnostik von Veränderungen des Drehvermögens und für die Kontrolle von Therapien.

Es hat sich gezeigt, daß mit der erfindungsgemäßen Vorrichtung Drehfähigkeitsmessungen an einem großen Krankengut mit einer Plus-/Minus-Abweichung von 2,9° möglich sind.

Die Maßnahme des Anspruchs 2 gestattet eine kontrollierte Drehbewegung des Kopfes, wobei natürlich im Hinblick auf unterschiedliche Hinterkopfformen noch nicht sichergestellt ist, daß die durch das Zusammenwirken von Hinterkopfform und Schalenform bedingte tatsächliche Drehachse exakt zusammenfällt mit dem anatomischen Drehachsensystem, das annähernd mit dem im obersten Halswirbel gelagerten Zahnfortsatz des zweiten Halswirbels zusammenfällt. Diese mögliche Abweichung läßt sich aber bei der Auswertung des angezeigten Bogens berücksichtigen, wenn man den zur Achse der Halswirbelsäule senkrechten Abstand des Zahnfortsatzes des zweiten Halswirbels von der Schreibspitze kennt.

Aus Gründen der einfachen Fertigung der Hinterkopflagerung wird die Maßnahme nach dem Anspruch 3 angewandt.

Gemäß den Lehren des Anspruchs 4 wird für eine definierte Lage der Untersuchungsperson zur Hinterkopflagerung gesorgt; die Lösbarkeit der Schale ist von Bedeutung im Hinblick auf die hygienische Handhabung der Schale und ggf. auf die raumsparende Zusammenlegbarkeit der Einrichtung. Die Verstellbarkeit in Längsrichtung der Halswirbelsäule dient der Anpassung an unterschiedliche Größen der Untersuchungsperson.

Die Maßnahme des Anspruchs 5 dient der einfachen Handhabung der Einrichtung insofern, als für jeden Untersuchungsvorgang der Beschriftungsbogen einfach und rasch ausgewechselt werden kann.

Die Maßnahme des Anspruchs 6 dient der reproduzierbaren Zuordnung von Schreibfläche und Hinterkopflagerung.

Die Maßnahme des Anspruchs 7 führt den Gedanken des Anspruchs 6 fort im Hinblick auf eine einfache Handhabung der Vorrichtung und eine raumsparende Aufbewahrung sowie eine raumsparende Transportmöglichkeit für die Vorrichtung. Diese Maßnahme wird durch die Lehren des Anspruchs 8 weitergebildet. Dabei kann das Gelenkband derart feststellbar sein, daß in Anpassung an unterschiedliche anatomische Gegebenheiten auch eine Abweichung der Schreibplatte von der Senkrechtstellung gegenüber der Trägerplatte bzw. Rückenauflageplatte möglich ist.

Der Anspruch 9 schafft eine einfache Ausführungsform für den Träger. Die Gesichtsmaske kann etwa nach Art einer Tauchermaske im Anlagebereich an das Gesicht der Untersuchungsperson ausgebildet sein, wobei dieser Anlagebereich einerseits so elastisch sein muß, daß er sich unterschiedlichen Gesichtspartien verschiedener Untersuchungspersonen ohne schmerzhafte Druckstellen anpaßt, andererseits aber so steif, daß eine annähernd definierte Lage der Maske gegenüber dem Kopf der Untersuchungsperson gewährleistet ist.

Die Maßnahme des Anspruchs 10 vereinfacht die Messung des senkrecht zur Achse der Halswirbelsäule gemessenen Abstandes zwischen dem Zahnfortsatz des zweiten Halswirbels und der Schreibspitze und beruht auf der anatomischen Tatsache, daß der Zahnfortsatz des zweiten Halswirbels annähernd auf der Verbindungslinie der Felsenbeinspitzen liegt.

Der Anspruch 11 gibt eine einfache Meßeinrichtung zur Ermittlung des senkrechten Abstandes der Verbindungslinie der Felsenbeinspitzen von der Schreibspitzenachse an.

Die Maßnahme des Anspruchs 12 sorgt dafür, daß der Schreibkontakt der Schreibspitze mit der Schreibfläche während einer Drehbewegung der Halswirbelsäule erhalten bleibt.

Die Maßnahme nach Anspruch 13 sorgt für die Anpaßbarkeit der Lage des Schreibelements an die Lage der Hinterkopflagerung gegenüber der Schreibfläche.

Die beiliegenden Figuren erläutern ein Ausführungsbeispiel der Erfindung:

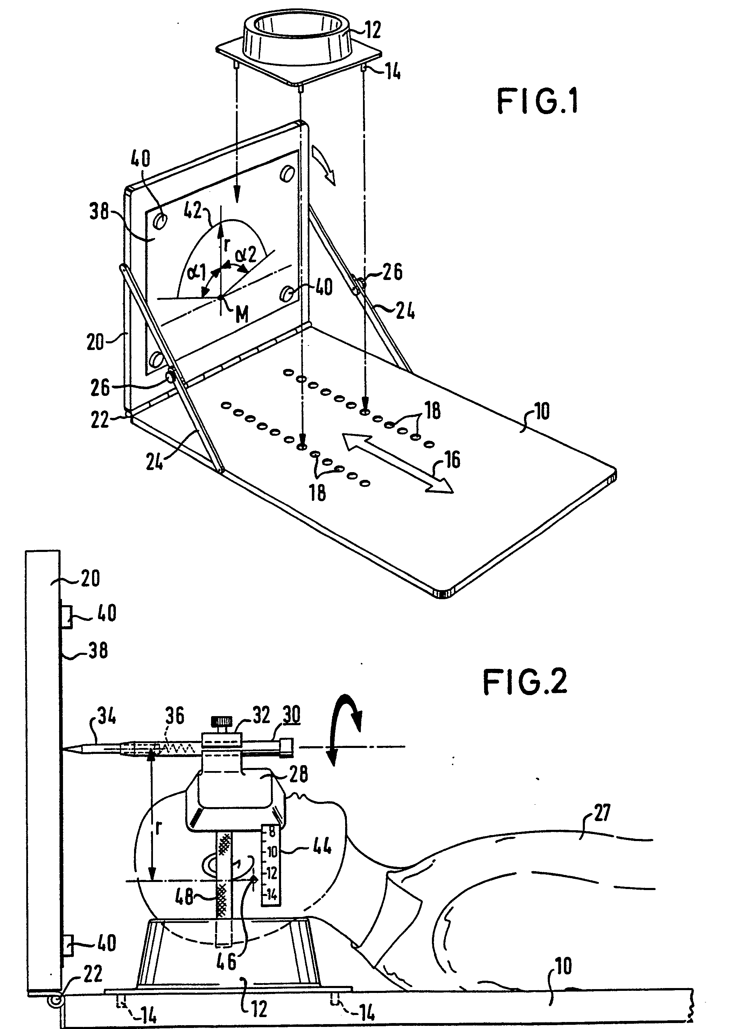

In Fig. 1 ist eine Trägerplatte mit 10 bezeichnet. Diese Trägerplatte kann selbst so groß ausgeführt sein, daß sie eine Liegefläche für die Untersuchungsperson liefert. Sie kann aber auch zur Befestigung an einer herkömmlichen Untersuchungsliege ausgebildet sein. Auf der Trägerplatte 10 kann eine als Hinterkopflagerung ausgebildete Schale 12 mittels Positionierungsstiften 14 in verschiedenen Lagen längs der Liegerichtung 16 positioniert werden, wobei die Positionierungsstifte 14 in Positionierungslöcher 18 zum Eingriff gebracht werden. An der Trägerplatte 10 ist eine Schreibplatte 20 mittels eines Scharniers 22 gelenkig befestigt, wobei die Scharnierachse parallel zur Ebene der Trägerplatte 10 und senkrecht zur Liegerichtung 16 verläuft. Die Schreibplatte 20 kann für Aufbewahrung und Transport nach Abnahme der Schale 12 von der Trägerplatte 10 an der Trägerplatte 10 beigeklappt werden und ist in ihrer in Fig. 1 dargestellten Betriebsstellung durch Gelenkbänder 24 feststellbar, die ihrerseits durch Klemmschrauben 26 immobilisiert werden können.

Fig. 2 zeigt eine Untersuchungsperson 27 auf der Trägerplatte 10 liegend, wobei der Hinterkopf in Untersuchungsstellung von der Schale 12 aufgenommen ist. Die Untersuchungsperson trägt eine durch ein Band 48 fixierte Gesichtsmaske 28 als Träger für ein Schreibelement 30. Die Achse des Schreibelements 30 ist annähernd parallel zur Achse der Halswirbelsäule. Das Schreibelement 30 ist durch eine Klemmvorrichtung 32 befestigt und in Längsrichtung verstellbar. Eine Schreibspitze 34 des Schreibelements 30 ist durch eine Feder 36 nach links vorgespannt. An der Schreibplatte 20 ist ein Beschriftungsbogen 38 durch Haftmagnete 40 fixiert.

Die Untersuchungsperson wird veranlaßt, den Kopf in der Schale 12 unter Erhaltung des Kontakts mit dieser soweit wie möglich zunächst nach der einen und dann nach der anderen Seite zu drehen. Dabei beschreibt die Schreibspitze 34 einen Bogen 42 auf dem Beschriftungsbogen 38.

An der Gesichtsmaske 28 ist beidseits je ein Maßband 44 befestigt, und zwar in solchem Abstand von der Symmetrieebene des Kopfes, daß die Maßbänder im wesentlichen frei nach unten hängen und den Kopf seitlich berühren. Mit Hilfe dieser Maßbänder kann der Abstand r der Verbindungslinie der Felsenbeinspitzen 46 von der Schreibelementachse bestimmt werden, wobei die Messung dann abgelesen wird, wenn an beiden Maßbändern 44 gleiche Längen gemessen werden. Die Maßbänder 44 sind derart geeicht, daß an ihnen unmittelbar der Abstand r in der jeweils gewünschten Längenmaßeinheit, beispielsweise in cm, abgemessen werden kann. Der Abstand r stellt annähernd den Abstand des im obersten Halswirbel gelagerten Zahnfortsatz des zweiten Halswirbels von der Achse der Schreibspitze 34 dar, und zwar annähernd senkrecht zur Achse der Halswirbelsäule gemessen.

Nach Ermittlung des Abstandes r wird dieser - wie in Fig. 1 dargestellt - auf den Beschriftungsbogen 38 übertragen, so daß sich ein Mittelpunkt M ergibt. Von dem Mittelpunkt M aus können nun Strahlen zu den Endpunkten des Bogens 42 gezogen werden, und diese Strahlen bilden mit der vertikalen Linie durch den Mittelpunkt M Winkel α1 und α2, welche der Drehfähigkeit der Halswirbelsäule nach beiden Richtungen entsprechen und beispielsweise in Winkelgraden gemessen werden. Es ist auch möglich, nach Ermittlung des Abstandes r einen Kreis zu ziehen, der dem Verlauf des Bogens 42 möglichst angenähert ist etwa in der Weise, daß die Abstände zwischen dem Kreis und dem Bogen über den ganzen Bogenverlauf minimal bleiben und dadurch den Mittelpunkt M festlegen, um danach wiederum von dem Mittelpunkt M aus die Strahlen zu den Endpunkten des gezeichneten Bogens 42 zu ziehen und die Winkel α1 und α2 zu ermitteln. Weiterhin ist es möglich, mit dem Radius r einen Kreis so zu legen, daß er die beiden Endpunkte des Bogens 42 tangiert und dann von dem so gewonnenen Mittelpunkt aus die Winke α1 und α2 zu bestimmen. Dabei ist es selbstverständlich notwendig immer die gleiche Ablesungsmethode anzuwenden.

**Patentansprüche**

1. Vorrichtung zur Erfassung der Drehfähigkeit der Halswirbelsäule gekennzeichnet durch eine Hinterkopflagerung (12) welche eine Drehbewegung des Kopfes gegenüber dem Oberkörper einer Untersuchungsperson (27) um die Achse der Halswirbelsäule gestattet, eine zur Achse der Halswirbelsäule senkrechte Schreibfläche (38) und einen an der Gesichtspartie der Untersuchungsperson zu befestigenden Träger (28) für ein Schreibelement (30) zum Aufzeichnen eines der Drehfähigkeit der Halswirbelsäule entsprechenden Bogens (42) auf der Schreibfläche (38).

2. Einrichtung nach Anspruch 1, dadurch gekennzeichnet, daß die Hinterkopflagerung (12) als eine der Wölbung eines durchschnittlichen Hinterkopfes angepaßte Schale (12) ausgebildet ist.

3. Einrichtung nach Anspruch 2, dadurch gekennzeichnet, daß die Schale eine im wesentlichen rotationssymmetrische Lagerfläche besitzt.

4. Einrichtung nach einem der Ansprüche 2 und 3, dadurch gekennzeichnet, daß die Schale (12) auf einer Rückenauflageplatte oder einer auf einer Rückenauflageplatte anzubringenden Trägerplatte (10) ggf. lösbar angebracht ist, ggf. in Längsrichtung der Halswirbelsäule verstellbar.

5. Einrichtung nach einem der Ansprüche 1 bis 4, dadurch gekennzeichnet, daß die Schreibfläche eine Schreibplatte (20) umfaßt, auf welcher ein Beschriftungsbogen (38) als Schreibfläche lösbar zu befestigen ist, beispielsweise durch Haftmagnete (40).

6. Einrichtung nach Anspruch 5, dadurch gekennzeichnet, daß die Schreibplatte (20) mit der Trägerplatte (10) oder der Rückenauflageplatte verbunden ist.

7. Einrichtung nach Anspruch 6, dadurch gekennzeichnet, daß die Schreibplatte (20) mit der Trägerplatte (10) oder der Rückenauflageplatte um eine zur Ebene der Trägerplatte bzw. Rückenauflageplatte parallele und zur Liegerichtung (16) senkrechte Achse schwenkbar durch ein Scharnier (22) verbunden ist, derart daß die Schreibplatte (20) zwischen einer zur Trägerplatte (10) bzw. Rückenauflageplatte parallelen Lager- und Transportstellung und einer zur Trägerplatte (10) bzw. Rückenauflageplatte senkrechten Arbeitsstellung verschwenkbar ist.

8. Einrichtung nach Anspruch 7, dadurch gekennzeichnet, daß die Schreibplatte (20) in ihrer Arbeitsstellung gegenüber der Trägerplatte (10) bzw. Rückenauflageplatte durch ein Gelenkband (24) feststellbar ist.

9. Einrichtung nach einem der Ansprüche 1 bis 8, dadurch gekennzeichnet, daß der Träger für das Schreibelement (30) als Gesichtsmaske (28) ausgebildet ist, welche durch ein um den Hinterkopf führendes Band (48) in der Betriebsstellung befestigbar ist.

10. Einrichtung nach einem der Ansprüche 1 bis 9, dadurch gekennzeichnet, daß an dem Träger (28) eine Meßeinrichtung (44) angebracht ist, welche den zur Achse der Halswirbelsäule senkrechten Abstand (r) der Schreibspitze (34) des Schreibelements (30) von der Verbindungslinie der Felsenbeinspitzen (46) der Untersuchungsperson zu ermitteln gestattet.

11. Einrichtung nach Anspruch 10, dadurch gekennzeichnet, daß die Meßeinrichtung von einem seitlich, vorzugsweise beidseitig an dem Träger (28) angebrachten Maßband (44) gebildet ist.

12. Einrichtung nach einem der Ansprüche 1 bis 11, dadurch gekennzeichnet, daß die Schreibspitze (34) des Schreibelements (30) gegenüber dem Träger (28) in Richtung der Achse der Halswirbelsäule gefedert ist.

13. Einrichtung nach einem der Ansprüche 1 bis 12, dadurch gekennzeichnet, daß das Schreibelement (30) an dem Träger (28) in Richtung der Achse der Halswirbelsäule verstellbar angeordnet ist.

**Claims**

1.) Device for detecting the rotatability of the cervical vertebral column, characterised by a mounting (12) for the back of the head which permits a rotating movement of the head in relation to the trunk of an examinee (27) about the axis of the cervical vertebral column, a writing surface (38) perpendicular to the axis of the cervical vertebral column and a carrier (28), for securing to the facial part of the examinee, for a writing element (30) for the recording of an arc (42), corresponding to the rotatability of the cervical vertebral column, on the writing surface (38).

2.) Device according to Claim 1, characterised in that the mounting (12) for the back of the head is formed as a dish (12) adapted to the curvature of an average head back.

3.) Device according to Claim 2, characterised in that the dish possesses a substanti1lly rotationally symmetrical support surface.

4.) Device according to one of Claims 2 and 3, characterised in that the dish (12) is fitted, possibly detachably, on a back rest panel or a carrier panel (10) for fitting on a back rest panel, possitly adjustably in the longitudinal direction of the cervical vertetral column.

5.) Device according to one of Claims 1 to 4, characterised in that the writing surface comprises a writing panal (20) on which an inscription sheat (38) can be fitted detachably, for example by attachment magnets (40), as writing surface.

6.) Device according to Claim 5, characterised in that the writing panel (20) is connected with the carrier panel (10) or the back rest panel.

7.) Device according to Claim 6, characterised in that the writing panel (20) is connected, by a hinge (22), with the carrier panel (10) or the back rest panel, for pivoting about an axis parallel to the plane of the carrier panel or back rest panel and perpendicular to the lying direction (16), in such a way that the writing panel (20) is pivotable between a storage and transport position parallel to the carrier panel (10) or back rest panel and a working position perpendicular to the carrier panel (10) or back rest panel.

8.) Device according to Claim 7, characterised in that the writing panel (20) is securable in its working position in relation to the carrier panel (10) or back rest panel by a hinge strap (24).

9.) Device according to one of Claims 1 to 8, characterised in that the carrier for the writing element (30) is formed as a face mask which can be secured in the operating position by a strap (48) leading round the back of the head.

10.) Device according to one of Claims 1 to 9, characterised in that on the carrier (28) there is fitted a measuring device (44) which permits of ascertaining the distance (r), perpendicular to the axis of the cervical vertebral column, of the writing tip (34) of the writing element (30) from the line of connection of the tips (46) of the petrous part of the temporal bones of the examinee.

11.) Device according to Claim 10, characterised in that the measuring device is formed by a measuring strip (44) fitted laterally, preferably on both sides, on the carrier (28).

12.) Device according to one of Claims 1 to 11, characterised in that the writing tip (34) of the writing element (30) is sprung in relation to the carrier (28) in the direction of the axis of the cervical vertebral column.

13.) Device according to one of Claims 1 to 12, characterised in that the writing element (30) is secured on the carrier (28) adjustably in the direction of the axis of the cervical vertebral column.

**Revendications**

1. Dispositif pour enregistrer la capacité de rotation de la partie cervicale de la colonne vertébrale, caractérisé par un appui nucal (12) autorisant un mouvement de rotation de la tête par rapport au tronc d'une personne examinée (27), autour de l'axe de la partie cervicale de la colonne vertébrale; une surface d'écriture (38) perpendiculaire à l'axe de la partie cervicale de la colonne vertébrale; et un support (28), devant être fixé à la région faciale de la personne examinée, pour un élément traceur (30) destiné à dessiner, sur la surface d'écriture (38), une courbe (42) correspondant à la capacité de rotation de la partie cervicale de la colonne vertébrale.

2. Dispositif selon la revendication 1, caractérisé par le fait que l'appui nucal (12) est réalisé sous la forme d'une écuelle (12) adaptée au bombement d'une nuque moyenne.

3. Dispositif selon la revendication 2, caractérisé par le fait que l'écuelle possède une surface de montage sensiblement à symétrie de rotation.

4. Dispositif selon l'une des revendications 2 et 3, caractérisé par le fait que l'écuelle (12) est éventuellement installée amoviblement sur une plaque d'appui dorsal ou sur une plaque de support (10) devant être placée sur une plaque d'appui dorsal, et est éventuellement réglable dans le sens longitudinal de la partie cervicale de la colonne vertébrale.

5. Dispositif selon 1 une des revendications 1 à 4, caractérisé par le fait que la surface d'écriture consiste en un panneau d écriture (20) sur lequel doit être fixée amoviblement, par exemple au moyen d'aimants de retenue (40), une feuille d'inscription (38) faisant office de surface d'écriture.

6. Dispositif selon la revendication 5, caractérisé par le fait que le panneau d'écriture (20) est relié à la plaque de support (10) ou à la plaque d'appui dorsal.

7. Dispositif selon la revendication 6, caractérisé par le fait que le panneau d'écriture (20) est relié à la plaque de support (10) ou à la plaque d'appui dorsal par l'intermédiaire d'une charnière (22), avec faculté de pivotement autour d'un axe parallèle au plan respectif de la plaque de support ou de la plaque d'appui dorsal et perpendiculaire à la direction (16) de la position couchée, de telle sorte que le panneau d'écriture (20) puisse accomplir un pivotement entre une position de stockage et de transport, respectivement parallèle à la plaque de support (10) ou à la plaque d'appui dorsal, et une position de travail respectivement perpendiculaire à la plaque de support (10) ou à la plaque d'appui dorsal.

8. Dispositif selon la revendication 7, caractérisé par le fait que le panneau d'écriture (20) peut être arrêté, par une barrette d'articulation (24), dans sa position de travail par rapport à la plaque de support (10) ou à la plaque d'appui dorsal, respectivement.

9. Dispositif selon l'une des revendications 1 à 8, caractérisé par le fait que le support de l'élément traceur (30) est réalisé sous la forme d'un masque facial (28) pouvant être fixé dans la position de service par l'intermédiaire d'un ruban (48) passant autour de la nuque.

10. Dispositif selon l'une des revendications 1 à 9, caractérisé par le fait qu'un dispositif mesureur (44), installé sur le support (28), permet de déterminer la distance (r) comprise, perpendiculairement à l'axe de la partie cervicale de la colonne vertébrale, entre la pointe d'écriture (34) de l'élément traceur (30) et la ligne reliant les pointes (46) des rochers de la personne examinée.

11. Dispositif selon la revendication 10, caractérisé par le fait que le dispositif mesureur est formé par un ruban mesureur (44) installé sur le côté, de préférence de part et d'autre du support (28).

12. Dispositif selon l'une des revendications 1 à 11, caractérisé par le fait que la pointe d'écriture (34) de l'élément traceur (30) est douée d'élasticité, par rapport au support (28), dans la direction de l'axe de la partie cervicale de la colonne vertébrale.

13. Dispositif selon l'une des revendications 1 à 12, caractérisé par le fait que l'élément traceur (30) est monté réglable sur le support (28), dans la direction de l'axe de la partie cervicale de la colonne vertébrale.

FIG.1

FIG.2